# EUROPEAN PATENT SPECIFICATION

(11) **EP 2 364 208 B1**
(45) Date of publication and mention of the grant of the patent: **14.01.2015**
(21) Application number: 09748865.4
(22) Date of filing: 27.10.2009
(51) Int. Cl.: B01J 23/68, C07C 67/055

(54) **SUPPORTED PALLADIUM-GOLD CATALYSTS AND PREPARATION OF VINYL ACETATE THEREWITH**
PALLADIUM-GOLD-TRÄGERKATALYSATOREN UND HERSTELLUNG VON VINYLACETAT DAMIT
CATALYSEURS PALLADIUM-OR SUR SUPPORT, ET PRÉPARATION D'ACÉTATE DE VINYLE AVEC CES CATALYSEURS

(30) Priority: 12.11.2008 US 291628
(43) Date of publication of application: 14.09.2011
(73) Proprietor: Lyondell Chemical Technology, L.P., Wilmington, DE 19803 (US)
(72) Inventor: SHAY, Daniel, Travis, Glen Mills Pennsylvania 19342 (US)
(74) Representative: Gaverini, Gaetano Luigi Attilio
(86) International application number: PCT/US2009/005828
(87) International publication number: WO 2010/056275

(56) References cited:
- EP-A1- 2 072 119
- EP-A2- 0 967 009
- US-A- 4 552 860
- US-A- 6 022 823
- US-A1- 2007 179 310

## Description

### FIELD OF THE INVENTION

The invention relates to a supported palladium-gold catalyst. More particularly, the invention relates to a supported palladium-gold catalyst that has increased catalytic activity and selectivity in acetoxylation.

### BACKGROUND OF THE INVENTION

Palladium-gold catalysts are known. They are used in acetoxylation. For instance, the oxidation of ethylene in the presence of a palladium-gold catalyst and acetic acid produces vinyl acetate, which is a useful monomer for the polymer industry. Acetoxylation is commonly performed by the vapor phase reaction using supported palladium-gold catalysts. Methods for supporting palladium-gold catalysts are known. In general, the method involves depositing a mixture of palladium and gold compounds onto a support and then reducing the palladium and gold to metals.

Palladium and gold are both precious metals. Therefore, many efforts have been made to increase the catalytic activity and reduce the amount of catalyst needed. For example, U.S. Pat. No. 6,022,823 teaches calcining the support impregnated with palladium and gold compounds prior to reducing the metals. The catalyst shows improved activity.

EP0967009 discloses a catalyst for use in the production of vinyl acetate which comprises (1) a catalyst support, (2) palladium, (3) an acid, (4) at least one acetic acid catalyst promoter and (5) at least one vinyl acetate promoter and/or co-promoter.

In US2007179310 a method for preparing supported palladium-gold catalysts is disclosed. The method comprises sulfating a titanium dioxide support, calcining the sulfated support, impregnating the calcined support with a palladium salt, a gold salt, and an alkali metal or ammonium compound, calcining the impregnated support, and reducing the calcined support.

US4552860 discloses a catalyst for use in a vapor phase process for the preparation of unsaturated aldehydes, carboxylic acids and mixtures thereof which comprises a noble metal promoted by a solid acid consisting of mixed metal oxides.

One challenge still facing the industry is that the supported palladium-gold catalyst has a low selectivity in acetoxylation. Due to the low selectivity, a large amount of ethylene is oxidized to carbon dioxide. Thus, it is important to the industry to increase the catalytic activity and selectivity of the supported palladium-gold catalysts.

### SUMMARY OF THE INVENTION

The invention is a catalyst. The catalyst comprises palladium and gold. The catalyst is supported on a support comprising titanium dioxide and tungsten trioxide. The support comprises from 75 wt% to 99 wt% of titanium dioxide and from 1 wt% to 25 wt% of tungsten trioxide. The invention includes a method for preparing the catalyst. The method comprises impregnating the support with a palladium compound and a gold compound. The impregnated support is calcined and then reduced to convert the palladium and gold compounds to metals. The invention also includes a method for preparing vinyl acetate with the catalyst of the invention. The method comprises oxidizing ethylene in the presence of acetic acid and the catalyst. The catalyst of the invention significantly improves the catalytic activity and the oxygen selectivity to the formation of vinyl acetate.

### DETAILED DESCRIPTION OF THE INVENTION

The invention is a catalyst. The catalyst comprises palladium and gold and is supported on a support comprising titanium dioxide and tungsten trioxide. The support comprises from 75 wt% to 99 wt% of titanium dioxide and from 1 wt% to 25 wt% of tungsten trioxide. More preferably, the support comprises from 80 wt% to 99 wt% of titanium dioxide and from 1 wt% to 20 wt% of tungsten trioxide. Most preferably, the support comprises from 80 wt% to 95 wt% of titanium dioxide and from 5 wt% to 20 wt% of tungsten trioxide. Particularly suitable supports are those which are commercially available, e.g., DT-52™ titania from Millennium Inorganic Chemicals, Inc. The catalyst of the invention comprises from 0.1 wt % to 3 wt % of palladium and from 0.1 wt % to 3 wt % of gold and has a weight ratio of palladium to gold within the range of 5:1 to 1:3. Preferably, the catalyst comprises 0.5 wt % to 1.5 wt % of palladium and 0.25 wt % to 0.75 wt % of gold and has a weight ratio of palladium to gold within the range of 2.5:11 to 1:1.5.

The support is impregnated with a palladium compound, a gold compound, and an optional alkali metal or ammonium compound. Any suitable impregnation method can be used. The support can be simultaneously or successively impregnated with a palladium compound, a gold compound, and an optional alkali metal or ammonium compound. Preferably, the impregnation is performed in solutions. Suitable palladium compounds include palladium chloride, sodium chloropalladite, palladium nitrate, palladium sulfate, the like, and mixtures thereof. Suitable gold compounds include auric chloride, tetrachloroauric acid, sodium tetrachloroaurate, the like, and mixtures thereof. Sodium tetrachloroaurate and palladium chloride or sodium chloropalladite are most commonly used. Suitable alkali metal or ammonium compounds include alkali metal or ammonium hydroxides, alkali metal or ammonium carbonates, alkali metal or ammonium bicarbonates, alkali metal or ammonium metasilicates, the like, and mixtures thereof.

One method to impregnate the support involves first treating the support with a solution of an alkali metal or ammonium compound. The support is then impregnated with a solution containing palladium and gold compounds. In another method, the impregnation with the palladium and gold solutions is carried out before treatment with the solution of the alkali metal or ammonium compound. In this procedure the pores of the support is essentially completely filled with the solution of palladium and gold compounds. Typically, this is accomplished by dropping the solution onto the support until incipient wetness is achieved. The support impregnated with the palladium and gold compounds is then contacted with the alkali metal or ammonium compound. A third method involves mixing the alkali or ammonium compound and palladium and gold compounds prior to contacting with the support. The contact with the support can be done by dropping or spraying the mixture onto the support until incipient wetness or by making a slurry of the support in the solution.

The impregnated support is preferably washed with water to remove alkali metal compounds such as chlorides formed during the impregnation and dried prior to calcination. The impregnated support is calcined, i.e., heated at an elevated temperature in a non-reducing atmosphere. Preferably, the calcination is performed under such a condition that a portion of the palladium and gold compounds are decomposed. More preferably, at least 10% of the palladium and gold compounds are decomposed during the calcination. Preferably, the calcination of the impregnated support is carried out at a temperature within the range of about 100°C to about 600°C. More preferably, the temperature is within the range of 100°C to 300°C. Most preferably, the temperature is within the range of 150°C to 250°C. Suitable non-reducing gases used for the calcination include inert or oxidizing gases such as helium, nitrogen, argon, neon, nitrogen oxides, oxygen, air, carbon dioxide, the like, and mixtures thereof. Preferably, the calcination is carried out in an atmosphere of nitrogen, oxygen, or air, or mixtures thereof.

After calcination, the impregnated support is reduced to convert the palladium and gold compounds to the corresponding metals. The reduction is performed by heating in the presence of a reducing agent. Suitable reducing agents include ammonia, carbon monoxide, hydrogen, hydrocarbons, olefins, aldehydes, alcohols, hydrazine, primary amines, carboxylic acids, carboxylic acid salts, carboxylic acid esters, the like, and mixtures thereof. Hydrogen, ethylene, propylene, alkaline hydrazine and alkaline formaldehyde are preferred reducing agents and ethylene and hydrogen are particularly preferred. Temperatures employed for the reduction can range from ambient up to about 600°C. Preferably, the reduction temperature is within the range of 300°C to 600°C. More preferably, the reduction temperature is within the range of 450°C to 550°C. The reduction results in the supported catalyst of the invention.

The catalyst of the invention has many uses. It can be used, for example, in the partial oxidation, hydrogenation, carbonylation, ammonia synthesis, selective hydrogenation, acetyloxylation, catalytic combustion or complete oxidation, three way catalysis, NOx removal, methanol synthesis, hydrogen peroxide synthesis, hydroformylation, alkylation and alkyl transfer, oxidative carbonylation, coupling of olefins with aromatics, and the preparation of methyl isobutyl ketone from acetone. The catalyst of the invention is particularly useful for the productions of vinyl acetate acetate are known. For instance, U.S. Pat. Nos. 3,743,607 and 3,775,342 teach how to prepare vinyl acetate using palladium-gold catalysts.

For the use in the productions of vinyl acetate and allyl acetate, the catalyst is preferably treated with a potassium compound such as potassium acetate. The potassium treatment can be done by mixing the catalyst with a potassium acetate solution, filtering, and drying the treated catalyst. In general, vinyl acetate can be made by the oxidation of ethylene in the presence of acetic acid and the catalyst. Allyl acetate can be made by a similar manner but using propylene rather than ethylene. I surprisingly found that the catalyst of the invention gives not only high catalytic activity but also high selectivity in acetoxylation.

The following examples merely illustrate the invention. Those skilled in the art will recognize many variations that are within the scope of the claims.

### EXAMPLE 1

### Palladium-Gold Catalyst On Titanium Dioxide-Tungsten Oxide Support

A support (30 grams, DT-52™, product of Millennium Inorganic Chemicals, Inc., containing 10 wt% of tungsten trioxide and 90 wt% of titanium dioxide) is calcined at 700°C for six hours. The calcined support is placed in a rotating glass dish with a baffle which aids in tumbling the carrier support during the metal impregnation. A solution containing 10.4 mL water, 294 mg NaAuCl₄ (Alfa), and 799 mg Na₂PdCl₄ (Alfa) is placed in a 250 mL beaker with a magnetic stir bar and is stirred for 2 minutes to ensure all the metal compounds are dissolved. To the solution is added 813 mg sodium bicarbonate (a product of Fisher) in 3 portions and stirring is continued for 3 minutes until no trace of gas evolution is observed. The solution is then added dropwise using a pipette to the support while it rotates in the glass dish at 35 rpm. Upon complete addition of the solution, 2 mL of deionized (DI) water is added to the beaker to rinse the sides of the beaker and then added to the rotating support dropwise. The glass dish is allowed to rotate for 15 minutes while being gently heated by a hot air gun before being placed in an 80°C oven for 24 hours to facilitate coordination of the Pd and Au to the support. Thereafter the material is removed from the oven and placed in a filter and rinsed with 2000 mL of 90°C DI water to remove sodium chloride. The filtrate is tested with silver nitrate and rinsing is continued until no trace of precipitate is observed. After washing, the impregnated support is placed in an 80°C oven and dried overnight. After drying, the catalyst is placed in a quartz tube and the tube is inserted into a three zone electric furnace and heated to 230°C under a 120 mL/min flow of dry air for 3 hours. After 3 hours the quartz tube is purged with nitrogen for 30 minutes and the temperature is increased to 500°C and held for 3 hours under a 120 mL/min flow of 5% hydrogen in helium. After the three hours, nitrogen is introduced and the temperature is decreased to 25°C. After cooling, the supported catalyst is submerged in a beaker filled with 200 mL of a 5 wt% /0.5 wt% potassium acetate /potassium hydroxide solution for 20 minutes. After 20 minutes, the solution is decanted and the beaker placed in an 80°C oven for 24 hours to dry. After drying, the supported catalyst is placed in a plastic bottle for storage.

The supported catalyst prepared above is tested in a continuous bench-scale vinyl acetate unit. Ethylene, acetic acid vapor, and oxygen are reacted over the supported catalyst to produce vinyl acetate. Reaction is run at a pressure range of 35 to 110 psig and a temperature range of 110 to 180°C. The reactants are fed downward through the catalyst bed. All feed gases are monitored by mass flow controllers. Acetic acid is fed by a metering pump. Ethylene, oxygen, nitrogen, and helium are premixed through a heat traced tube and fed to the reactor. Oxygen is supplied from a blended cylinder consisting of 20% oxygen, 10% nitrogen, and 70% helium. Acetic acid is fed separately through a heated line, to ensure complete vaporization, and combined with the gas feed at the top of the reactor. The pressure of the effluent is reduced and the effluent is sent to an analysis system (Gas Chromatography) via heat traced lines. After the gas stream had been analyzed it is sent to a knock out drum and collected as organic waste. Non-condensables are sent directly to the vent stack of the fume hood. The reactor is immersed in and heated by an air fluidized sand bath. Pressure is sensed by an electronic transmitter at the reactor inlet. The conversion, selectivity, and recovery data are obtained from known amounts of metered feeds and the GC analysis of the reactor effluent. Nitrogen from the oxygen/nitrogen/helium blend serves as an internal standard. The results are listed in Table 1 and Table 2. The oxygen selectivity is the ratio of oxygen converted to vinyl acetate/total oxygen consumed.

### COMPARATIVE EXAMPLE 2

### CONVENTIONAL PALLADIUM-GOLD CATALYST SUPPORTED ON TITANIUM DIOXIDE

The general procedure of Example 1 is followed but a titanium dioxide (DT-51™, product of Millennium Inorganic Chemicals, Inc.) is used. The supported catalyst is used for preparing vinyl acetate following the procedure in Example 1. The results are listed in Table 1 and Table 2.

Table 1 compares the catalytic activity of the supported palladium-gold catalyst of the invention with the conventional catalyst supported on titanium dioxide at essentially the same oxygen selectivity. The results indicate that when the sand bath temperature is kept the same (123°C), the reactor internal temperature for the supported catalyst of the invention is about 13°C higher than that for the conventional catalyst. This temperature increase suggests that the supported catalyst of the invention is an order of magnitude more reactive than that of the conventional catalyst.

**TABLE 1**

| COMPARISON OF CATALYTIC ACTIVITY OF THE SUPPORTED CATALYST OF THE INVENTION AND CONVENTIONAL CATALYST* | | | | | |
|---|---|---|---|---|---|
| Catalyst | Sand Bath Temp (°C) | Internal Temp (°C) | Oxygen Conversion (%) | Oxygen Selectivity (%) | Productivity (Lbs VA/hr/100 Lbs cat.) |
| Invention (Ex.1) | 123 | 137.8 | 55.5% | 79.0% | 37.4 |
| Conventional (C.2) | 123 | 124.3 | 38.4% | 80.7% | 26.5 |

| | | | | | |
|---|---|---|---|---|---|
| * The data presented in this Table is a ten-hour average after a thirty-hour catalyst break-in. At this point, the reactor internal temperature remains essentially constant. | | | | | |

Table 2 compares the oxygen selectivity of the supported catalyst of the invention with the conventional catalyst at essentially the same catalytic activity. The same catalytic activity is achieved by rising the sand bath temperature for the conventional catalyst to an extent that the conventional catalyst shows the same catalytic activity as the catalyst of the invention. The results indicate that the supported catalyst of the invention has significantly improved oxygen selectivity.

**TABLE 2**

| COMPARISON OF SELECTIVITY OF THE SUPPORTED CATALYST OF THE INVENTION AND CONVENTIONAL CATALYST | | |
|---|---|---|
| Reaction time (hr) | Oxygen Selectivity Invention Catalyst (Ex.1) | Oxygen Selectivity Conventional Catalyst (C.2) |
| 1 | 77% | 66% |
| 2 | 78% | 64% |
| 3 | 79% | 64% |
| 4 | 79% | 65% |
| 5 | 79% | 66% |
| 7 | 79% | 66% |
| 8 | 80% | 66% |
| 9 | 79% | 67% |
| 10 | 80% | 67% |
| 11 | 79% | 67% |
| 12 | 80% | 68% |
| 13 | 79% | 68% |
| 14 | 79% | 68% |
| 15 | 79% | 68% |
| 16 | 80% | 67% |
| 17 | 80% | 68% |
| 18 | 79% | 68% |
| 19 | 79% | 68% |
| 20 | 80% | 68% |
| 21 | 80% | 69% |
| 22 | 80% | 69% |
| 23 | 80% | 69% |
| 24 | 78% | 69% |

## Claims

1. A catalyst comprising from 0.1 wt % to 3 wt % of palladium, from 0.1 wt % to 3 wt % of gold, and a support comprising from 75 wt% to 99 wt% of titanium dioxide and from 1 wt% to 25 wt% of tungsten trioxide, wherein the weight ratio of palladium to gold is of 5:1 to 1:3.

2. The catalyst of claim 1 wherein the support comprises from 80 wt% to 99 wt% of titanium dioxide and from 1 wt% of 20 wt% of tungsten trioxide.

3. The catalyst of claim 2 wherein the support comprises from 80 wt% to 95 wt% of titanium dioxide and from 5 wt% to 20 wt% of tungsten trioxide.

4. A method for preparing a catalyst according to any one the preceding claims, said method comprising:
(a) impregnating a support comprising titanium dioxide and tungsten trioxide with a palladium compound, a gold compound, and an optional alkali metal or ammonium compound;
(b) calcining the impregnated support; and
(c) reducing the palladium and gold compounds to metals.

5. The method of claim 4 wherein the palladium compound is selected from the group consisting of palladium chloride, sodium chloropalladite, palladium nitrate, palladium sulfate, and mixtures thereof, and the gold compound is selected from the group consisting of auric chloride, tetrachloroauric acid, sodium tetrachloroaurate, and mixtures thereof.

6. The method of claim 4 wherein the support comprises from 85 wt% to 95 wt% of titanium dioxide and from 5 wt% to 15 wt% of tungsten trioxide.

7. The method of claim 4 wherein the calcination is performed at a temperature within the range of 100°C to 600°C.

8. The method of claim 4 wherein the reduction is performed at a temperature within the range of 300°C to 600°C in the presence of hydrogen.

9. A method for preparing vinyl acetate comprising oxidizing ethylene in the presence of acetic acid and a catalyst according to claims 1 to 3.

## Patentansprüche

1. Katalysator umfassend 0,1 Gew.-% bis 3 Gew.-% Palladium, 0,1 Gew.-% bis 3 Gew.-% Gold und einen Träger, der 75 Gew.-% bis 99 Gew.-% Titandioxid und 1 Gew.-% bis 25 Gew.-% Wolframtrioxid umfasst, wobei das Gewichtsverhältnis von Palladium zu Gold 5:1 bis 1:3 beträgt.

2. Katalysator nach Anspruch 1, wobei der Träger 80 Gew.-% bis 99 Gew.-% Titandioxid und 1 Gew.-% bis 20 Gew.-% Wolframtrioxid umfasst.

3. Katalysator nach Anspruch 2, wobei der Träger 80 Gew.-% bis 95 Gew.-% Titandioxid und 5 Gew.-% bis 20 Gew.-% Wolframtrioxid umfasst.

4. Verfahren zur Herstellung eines Katalysators nach einem der vorhergehenden Ansprüche, wobei das Verfahren Folgendes umfasst:
(a) das Imprägnieren eines Trägers, der Titandioxid und Wolframtrioxid umfasst, mit einer Palladiumverbindung, einer Goldverbindung und einer wahlweisen Alkalimetall- oder Ammoniumverbindung;
(b) das Calcinieren des imprägnierten Trägers; und
(c) das Reduzieren der Palladium- und Goldverbindungen zu Metallen.

5. Verfahren nach Anspruch 4, wobei die Palladiumverbindung aus der Gruppe ausgewählt ist bestehend aus Palladiumchlorid, Natriumchlorpalladit, Palladiumnitrat, Palladiumsulfat und Mischungen davon und die Goldverbindung aus der Gruppe ausgewählt ist bestehend aus Goldchlorid, Tetrachlorgoldsäure, Natriumtetrachloroaurat und Mischungen davon.

6. Verfahren nach Anspruch 4, wobei der Träger 85 Gew.-% bis 95 Gew.-% Titandioxid und 5 Gew.-% bis 15 Gew.-% Wolframtrioxid umfasst.

7. Verfahren nach Anspruch 4, wobei das Calcinieren bei einer Temperatur innerhalb des Bereichs von 100°C bis 600°C durchgeführt wird.

8. Verfahren nach Anspruch 4, wobei die Reduktion bei einer Temperatur innerhalb des Bereichs von 300°C bis 600°C in Gegenwart von Wasserstoff durchgeführt wird.

9. Verfahren zur Herstellung von Vinylacetat, umfassend das Oxidieren von Ethylen in Gegenwart von Essigsäure und einem Katalysator nach einem der Ansprüche 1 bis 3.

## Revendications

1. Catalyseur comprenant 0,1% en poids à 3% en poids de palladium, 0,1% en poids à 3% en poids d'or et un support comprenant 75% en poids à 99% en poids de dioxyde de titane et 1% en poids à 25% en poids de trioxyde de tungstène, dans lequel le rapport pondéral du palladium à l'or vaut 5:1 à 1:3.

2. Catalyseur selon la revendication 1, dans lequel le support comprend 80% en poids à 99% en poids de dioxyde de titane et 1% en poids à 20% en poids de trioxyde de tungstène.

3. Catalyseur selon la revendication 2, dans lequel le support comprend 80% en poids à 95% en poids de dioxyde de titane et 5% en poids à 20% en poids de trioxyde de tungstène.

4. Procédé de préparation d'un catalyseur selon l'une quelconque des revendications précédentes, ledit procédé comprenant :
(a) l'imprégnation d'un support comprenant du dioxyde de titane et du trioxyde de tungstène par un composé de palladium, un composé d'or et un composé facultatif de métal alcalin ou d'ammonium ;
(b) la calcination du support imprégné ; et
(c) la réduction des composés de palladium et d'or en métaux.

5. Procédé selon la revendication 4, dans lequel le composé de palladium est choisi dans le groupe constitué par le chlorure de palladium, le chloropalladite de sodium, le nitrate de palladium, le sulfate de palladium et leurs mélanges, et le composé d'or est choisi dans le groupe constitué par le chlorure aurique, l'acide tétrachloroaurique, le tétrachloroaurate de sodium et leurs mélanges.

6. Procédé selon la revendication 4, dans lequel le support comprend 85% en poids à 95% en poids de dioxyde de titane et 5% en poids à 15% en poids de trioxyde de tungstène.

7. Procédé selon la revendication 4, dans lequel la calcination est réalisée à une température dans la plage de 100°C à 600°C.

8. Procédé selon la revendication 4, dans lequel la réduction est réalisée à une température dans la plage de 300°C à 600°C en présence d'hydrogène.

9. Procédé de préparation d'acétate de vinyle comprenant l'oxydation d'éthylène en présence d'acide acétique et d'un catalyseur selon les revendications 1 à 3.
